(19) Europäisches Patentamt
European Patent Office
Office européen des brevets

(11) **EP 3 325 631 B1**

(12) **EUROPÄISCHE PATENTSCHRIFT**

(45) Veröffentlichungstag und Bekanntmachung des Hinweises auf die Patenterteilung:
**03.09.2025 Patentblatt 2025/36**

(21) Anmeldenummer: **16741595.9**

(22) Anmeldetag: **15.07.2016**

(51) Internationale Patentklassifikation (IPC):
**C12N 15/863** (2006.01) **A61P 37/04** (2006.01)
**A61K 39/00** (2006.01)

(52) Gemeinsame Patentklassifikation (CPC):
**A61K 39/00; A61P 37/04; C12N 15/86;**
A61K 2039/5154; C12N 2710/24222;
C12N 2710/24243; C12N 2830/34; C12N 2830/60;
Y02A 50/30

(86) Internationale Anmeldenummer:
**PCT/EP2016/066926**

(87) Internationale Veröffentlichungsnummer:
**WO 2017/013023 (26.01.2017 Gazette 2017/04)**

(54) **REKOMBINANTER ORF-VIRUS-VEKTOR**

RECOMBINANT ORF-VIRUS VECTOR

VECTEUR DE VIRUS ORF RÉCOMBINÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.07.2015 DE 102015111756**

(43) Veröffentlichungstag der Anmeldung:
**30.05.2018 Patentblatt 2018/22**

(60) Teilanmeldung:
**25156960.4 / 4 545 094**

(73) Patentinhaber: **Prime Vector Technologies GmbH 72070 Tübingen (DE)**

(72) Erfinder:
• **RZIHA, Hans-Joachim 72074 Tuebingen (DE)**
• **AMANN, Ralf 72070 Tuebingen (DE)**

(74) Vertreter: **Graf von Stosch Patentanwaltsgesellschaft mbH Triftstraße 5 80538 München (DE)**

(56) Entgegenhaltungen:
• HANNS-JOACHIM RZIHA ET AL: "Genomic Characterization of Orf Virus Strain D1701-V (Parapoxvirus) and Development of Novel Sites for Multiple Transgene Expression", VIRUSES, vol. 11, no. 2, 30 January 2019 (2019-01-30), pages 127, XP055557139, DOI: 10.3390/v11020127
• RZIHA H-J ET AL: "Generation of recombinant parapoxviruses: non-essential genes suitable for insertion and expression of foreign genes", JOURNAL OF BIOTECHNOLOGY, ELSEVIER, AMSTERDAM NL, vol. 83, no. 1-2, 29 September 2000 (2000-09-29), pages 137 - 145, XP004212161, ISSN: 0168-1656, DOI: 10.1016/S0168-1656(00)00307-2
• AMANN RALF ERNST: "Orf Virusvektor: Entwicklung neuer Selektionsstrategien, Identifikation neuer Fremdgen- Insertionsorte sowie Herstellung, Charakterisierung und Wirksamkeit eines rekombinanten Tollwutimpfstoffs", DISSERTATION, 1 January 2014 (2014-01-01), pages 1 - 102, XP093026596, Retrieved from the Internet <URL:https://publikationen.uni-tuebingen.de/xmlui/handle/10900/52928> [retrieved on 20230223]

EP 3 325 631 B1

- G. DELHON ET AL: "Genomes of the Parapoxviruses Orf Virus and Bovine Papular Stomatitis Virus", JOURNAL OF VIROLOGY, vol. 78, no. 1, 1 January 2004 (2004-01-01), US, pages 168 - 177, XP055579007, ISSN: 0022-538X, DOI: 10.1128/JVI.78.1.168-177.2004
- FRIEDERICHS SCHIRIN ET AL: "Comparative and retrospective molecular analysis of Parapoxvirus (PPV) isolates", VIRUS RESEARCH, vol. 181, 27 December 2013 (2013-12-27), pages 11 - 21, XP028613887, ISSN: 0168-1702, DOI: 10.1016/J.VIRUSRES.2013.12.015
- ZHANGYONG NING ET AL: "Generation of recombinant Orf virus using an enhanced green fluorescent protein reporter gene as a selectable marker", BMC VETERINARY RESEARCH, BIOMED CENTRAL, LONDON, GB, vol. 7, no. 1, 22 December 2011 (2011-12-22), pages 80, XP021119029, ISSN: 1746-6148, DOI: 10.1186/1746-6148-7-80
- JOANNE L TAN ET AL: "Development of orf virus as a bifunctional recombinant vaccine: Surface display ofantigen EG95 by fusion to membrane structural proteins", VACCINE, ELSEVIER LTD, GB, vol. 30, no. 2, 28 October 2011 (2011-10-28), pages 398 - 406, XP028348290, ISSN: 0264-410X, [retrieved on 20111107], DOI: 10.1016/J.VACCINE.2011.10.079
- JÖRG ROHDE ET AL: "New Orf Virus (Parapoxvirus) Recombinant Expressing H5 Hemagglutinin Protects Mice against H5N1 and H1N1 Influenza A Virus", PLOS ONE, vol. 8, no. 12, 20 December 2013 (2013-12-20), pages e83802, XP055300959, DOI: 10.1371/journal.pone.0083802
- AMANN R ET AL: "A new rabies vaccine based on a recombinant ORF virus (parapoxvirus) expressing the rabies virus glycoprotein", JOURNAL OF VIROLOGY (ONLINE), AMERICAN SOCIETY FOR MICROBIOLOGY, US, vol. 87, no. 3, 1 February 2013 (2013-02-01), pages 1618 - 1630, XP002753921, ISSN: 1098-5514, [retrieved on 20121121], DOI: 10.1128/JVI.02470-12
- T. FISCHER ET AL: "Novel Recombinant Parapoxvirus Vectors Induce Protective Humoral and Cellular Immunity against Lethal Herpesvirus Challenge Infection in Mice", JOURNAL OF VIROLOGY, vol. 77, no. 17, 1 September 2003 (2003-09-01), pages 9312 - 9323, XP055005357, ISSN: 0022-538X, DOI: 10.1128/JVI.77.17.9312-9323.2003
- LI WEI ET AL: "Comparative genomic sequence analysis of Chinese orf virus strain NA1/11 with other parapoxviruses", ARCHIVES OF VIROLOGY, SPRINGER WIEN, AT, vol. 160, no. 1, 11 November 2014 (2014-11-11), pages 253 - 266, XP035419843, ISSN: 0304-8608, [retrieved on 20141111], DOI: 10.1007/S00705-014-2274-1
- M. BUTTNER ET AL: "Parapoxviruses: From the Lesion to the Viral Genome", JOURNAL OF VETERINARY MEDICINE. SERIES B - ZENTRALBLATT FUER VETERINAERMEDIZIN. REINE B., vol. 49, no. 1, 1 February 2002 (2002-02-01), DE, pages 7 - 16, XP055300947, ISSN: 0931-1793, DOI: 10.1046/j.1439-0450.2002.00539.x

Bemerkungen:

- Das gesamte Dokument mit Referenztabelle(n) und Sequenzliste(n) kann von der Webseite des EPA heruntergeladen werden
- Die Akte enthält technische Angaben, die nach dem Eingang der Anmeldung eingereicht wurden und die nicht in dieser Patentschrift enthalten sind.

# EP 3 325 631 B1

**Beschreibung**

Die vorliegende Erfindung betrifft einen rekombinanten Orf-Virus-Vektor, eine Zelle, die den rekombinanten Orf-Virus-Vektor enthält, eine den erfindungsgemäßen rekombinanten Orf-Virus-Vektor und/oder die erfindungsgemäße Zelle enthaltende Zusammensetzung, die Verwendung des erfindungsgemäßen rekombinanten Orf-Virus-Vektors zur Expression von einem Fremdgen sowie ein Nukleinsäuremolekül codierend für einen Orf-Virus-Vektor-Promotor.

Virale Vektoren werden in der Biotechnologie dafür verwendet, um genetisches Material in Zielzellen zu schleusen. Das eingeschleuste genetische Material codiert dabei häufig für Fremdgene, die der Herstellung eines rekombinanten Proteins dienen. Virale Vektoren stellen deshalb eine bedeutende Plattformtechnologie insbesondere zur Herstellung von rekombinanten Impfstoffen dar, die neben der klassischen Prävention von Infektionskrankheiten zunehmend auch zur Entwicklung von neuen, innovativen Therapiekonzepten, wie bspw. der therapeutischen Tumorimmunisierung, eingesetzt werden.

Das Orf-Virus (ORFV) gehört zu der Familie der Pockenviren und verfügt über eine Vielzahl von Eigenschaften, welche es zur Herstellung von rekombinanten Impfstoffen interessant macht und gegenüber anderen Technologien präferiert. Es stellt den Prototyp der Gattung der Parapockenviren dar und ist der Familie der *Poxviridae* zuzuordnen. ORFV sind behüllte, komplexe dsDNA-Viren von wollknäuelartiger Morphologie und einer mittleren Größe von ca. 260 x 160 nm. Sie besitzen ein lineares, GC-reiches, ca. 130 bis 150 kbp großes DNA-Genom, dessen zentraler Bereich beidseitig von ITR-Regionen ("inverted terminal repeats") abgegrenzt wird und in einer Haarnadelstruktur endet, welche beide DNA-Einzelstränge kovalent miteinander verknüpft. Im zentralen Bereich des Genoms befinden sich überwiegend Gene, die hauptsächlich für die virale Replikation und Morphogenese essentiell und innerhalb der Pockenviren hochkonserviert sind. Dagegen befinden sich in den ITR-Regionen sog. nicht-konservierte Virulenzgene, welche maßgeblich das Wirtsspektrum, die Pathogenität und die Immunmodulation bestimmen und somit das Virus charakterisieren.

Die virale Replikation ist bei Pockenviren auf das Zytoplasma restringiert und beginnt mit der Bindung des Virus an die Oberfläche der Wirtszelle. Nach der Fusion werden das sog. virale Core, ein Proteinkern, welches das virale Genom, frühe virale mRNA sowie virale Transkriptionsfaktoren (TF) beinhaltet, in das Zytoplasma freigesetzt. Nachfolgend beginnt die frühe virale Genexpression, in der unter Kontrolle von poxvirusspezifischen frühen ("early") Promotoren virale mRNA synthetisiert wird. Nach Auflösung der Core-Struktur wird die virale DNA im Zytoplasma freigesetzt. Im Gegensatz zu der Transkription von "early"-Genen, die ausschließlich unter Kontrolle viraler TF verläuft, ist die nachfolgende "intermediate" und "late" Gentranskription auf die Mithilfe zellulärer TF angewiesen. Somit benötigt die poxvirale Expression früher Gene, im Unterschied zur Expression von "intermediate"- und "late"-Genen, weder die Replikation viraler DNA noch eine Virusproduktion.

Das ORFV zeichnet sich durch einen sehr engen natürlichen Wirtsbereich aus, der Schaf und Ziege umfasst. Infektionen erfolgen über Läsionen der Haut, die das Eindringen des Virus ermöglichen. Die Vermehrung des dermatropen Virus ist anschließend auf regenerative Keratinozyten begrenzt, wodurch eine kontagiöse Dermatitis oder *Ecthyma contagiosum* ausgelöst wird. Diese in der Regel mild verlaufende, selbstlimitierende Infektion manifestiert sich als lokal begrenzte Haut- bzw. Schleimhautläsion, wobei die durch die massive Infiltration polymorphkerniger Lymphozyten ausgelöste Pustelbildung hauptsächlich an Maul und Euter auftritt. Die Läsion heilt nach ca. 4 bis 6 Wochen ohne Narbenbildung ab. Ein langanhaltender Immunschutz wird trotz der starken Immunantwort nicht ausgebildet, so dass eine Reinfektion bereits nach wenigen Wochen möglich ist, wenngleich klinische Symptome und Virusproduktion deutlich vermindert sind. Eine systemische Verbreitung oder Virämie wurde für ORFV nicht beobachtet; auch nicht nach experimenteller intravenöser Injektion hoher Dosen infektiöser Viren.

ORFV gilt als zoonotischer Erreger und ist in seltenen Fällen über die verletzte Haut auch auf den Menschen übertragbar. Nach der Infektion kommt es zu lokal begrenzten modulären Schwellungen, die sich zumeist auf Finger und Hände beschränken, sowie vereinzelt zu Schwellungen der Lymphknoten und Fieber. Für gewöhnlich ist der Verlauf harmlos, frei von Komplikationen und heilt innerhalb von drei bis acht Wochen ohne klinische Nachwirkungen vollständig ab. Bei immunsupprimierten Personen wurden auch schwerere Infektionsverläufe beobachtet, die jedoch nach Behandlung mit Virustatika, wie Cidofovir oder Imiquimod, vollständig verheilten.

ORFV ist für die Herstellung von rekombinanten Impfstoffen interessant. Im Vergleich zu Orthopockenviren zeichnet sich ORFV durch einen sehr engen natürlichen Wirtstropismus aus, der Schaf und Ziege umfasst. Damit kann im Menschen eine durch eine natürliche Infektion verursachte, inhibierende "Präimmunität" gegen den Vektor, wie sie bei den gängisten viralen Vektoren der Vaccinia- und Adenoviren beobachtet wird, nahezu ausgeschlossen werden. Des Weiteren ermöglicht die außergewöhnlich schwache und kurzlebige ORFV-spezifische Vektorimmunität sehr effektive Booster- und/oder Auffrischimpfungen bzw. Immunisierungen mit ORFV-basierten Impfstoffen, die sich gegen weitere Erreger richten.

Die Verabreichung von ORFV führt in permissiven, aber auch nicht-permissiven Wirten zu einer starken immunstimulierenden Reaktion, die sich durch eine ausgeprägte Induktion angeborener Immunmechanismen und der Freisetzung von Interferonen, Zytokinen und Chemokinen auszeichnet. Kurz nach der Immunisierung akkumulieren dendritische Zellen an der Injektionsstelle und leiten anschließend durch Aktivierung von T- und B-Zellen eine spezifische adaptive Immunantwort ein. Im Gegensatz zu Impfstoffen aus inaktivierten Viren oder Lebendvektoren, die meist eine humorallastige Immunantwort induzieren, ist die balancierte Induktion der zellulären und der humoralen Immunantwort nach Immunisierung mit rekombinanten ORFV ein entscheidender Vorteil. Zugleich macht es den Einsatz von Adjuvanzien, die zu unerwünschten Nebenwirkungen und Entzündungsreaktionen führen können, überflüssig. Als weitere Vorteile sind zudem die Möglichkeit der standardisierten Herstellung der rekombinanten Impfstoffe in permanenten Zelllinien ohne den Einsatz von Antibiotika, sowie der Verzicht auf eine Herstellung im Hühnerei (wachsende Anzahl an Eiweiß- und Antibiotika-Unverträglichkeiten) zu nennen.

Ein im Veterinärbereich zugelassener, attenuierter ORFV-Impfstoff trägt die Bezeichnung D1701, entsprechend dem gleichlautenden ORFV-Stamm. Dieser in inaktivierter Form unter dem Handelsnamen Baypamun (Bayer) bzw. Zylexis (Pfizer) bekannte Impfstoff wurde ursprünglich durch die Isolierung eines Wildtypvirus aus dem Schaf und anschließender Adaptierung infolge mehrfacher Passagierung in Rindernierenkulturzellen gewonnen. Es folgte eine weitere Adaptierung in Vero-Zellen (African green monkey kidney cells), was zum Erhalt des ORFV-Vektors D1701-V führte. D1701-V ist weiter attenuiert und ruft selbst in immunsupprimierten Schafen nur asymptotische Infektionen hervor.

Die bisherigen rekombinanten ORFV-Vektoren wählen als Insertionsort den VEGF-Lokus. Dies bietet nach derzeitigen Erkenntnissen den vermeintlichen Vorteil, dass durch die Eliminierung des unter der Kontrolle eines poxvirus-spezifischen "early"-Promotors stehenden und als Virulenzfaktor geltenden vegf-e Gens eine weitere Attenuierung des Vektors erfolgt. Inzwischen wurden mehrere ORFV-basierte, rekombinante Impfstoffe hergestellt und im Tiermodell untersucht. Derzeit werden rekombinante ORFV-Impfstoffe gegen unterschiedliche Infektionskrankheiten wie beispielsweise gegen die Aujeszky'sche Krankheit, Tollwut, Borna'sche Erkrankung, Influenza oder die klassische Schweinepest eingesetzt.

Die gute immunstimulierende und prophylaktische nutzbare Wirkung rekombinanter Orf-Viren konnte in den letzten Jahren durch die Etablierung einer Reihe von Impfstoffen bzw. Vakzinen gegen unterschiedliche virale Infektionskrankheiten gezeigt werden. Ein Überblick über die Verwendung von rekombinanten Pockenviren, einschließlich ORFV, zur Herstellung von rekombinanten Proteinen findet sich in Rziha et al. (2000), Generation of recombinant parapoxviruses: non-essential genes suitable for insertion and expression of foreign genes, Journal or Biotechnology Vol. 83, Seiten 137-145, und Büttner und Rziha (2002), Parapoxviruses: From the Lesion to the Viral Genome, J. Vet. Med. B. Vol. 49, Seiten 7-16.

Der eingeschränkte Einsatz bisheriger rekombinanter ORFV-Vektoren erklärt sich v.a. durch die langwierige Selektionsprozedur. Insbesondere war bisher eine Herstellung von polyvalenten Impfstoffen nicht möglich. Ferner weisen die bekannten rekombinanten ORFV-Vektoren keine gezielt regulierbare Expression der Fremdgene auf.

Ning et al., 2011 beschreiben ein schnelles Verfahren zur Herstellung rekombinanter Orf-Viren (ORFV) basierend auf dem *enhanced green fluorescent protein* (EGFP) Reportergen als selektivem Marker (Ning et al., BMC Veterinary Research, Generation of recombinant Orf virus using an enhanced green fluorescent protein reporter gene as a selectable marker, 2011, 7:80, 1-13). Tan et al., 2011 untersuchen Strategien einer Expression des immunogenen *Echinococcus granulosus* Peptids EG95 durch ORFV mit dem Ziel, eine rekombinante bivalente Vakzine zu entwickeln (Tan et al., Vaccine, Development of orf virus as a bifunctional recombinant Vaccine: Surface display of Echinococcus granulosus antigen EG95 by fusion to membrane structural proteins, 2011, 30, 398-406).

Vor diesem Hintergrund ist es eine der Erfindung zugrundeliegende Aufgabe, einen neuen rekombinanten ORFV-Vektor, wie in den Ansprüchen definiert ist, bereitzustellen, mit dem die Nachteile der bekannten ORFV-Vektoren und Vektorsysteme reduziert oder vermieden werden.

Die hier beschriebenen Insertionsloci (IL) 1, 2 und 3 sind im ORFV-Genom in folgenden Regionen lokalisiert sind:

| | IL 1 | IL 2 | IL 3 |
|---|---|---|---|
| Restriktionsfragment | Hind*III*-Fragment C, Kpn*I*-Fragment G, Bam*HI*-Fragment C/G, Eco*RI*-Fragment B | Hind*III*-Fragment I/J, Kpn*I*-Fragment B, Bam*HI*-Fragment A, Eca*RI*-Fragment A/E | Hind*III*-Fragment G/D, Kpn*I*-Fragment B, Bam*HI*-Fragment A, Eca*RI*-Fragment D |
| und/oder | | | |
| Gen/OLR | 006, 007 (dUTPase), 008 (G1L-Ank), 009 (G2L) | 102, 103 | 114, 115, 116, 117 (GIF) |
| und/oder | | | |
| Nukleotidposition | nt 500 $\pm$ 100 bis | nt 5.210 $\pm$ 100 bis | nt 15.660 $\pm$ 100 bis |

(fortgesetzt)

|  | IL 1 | IL 2 | IL 3 |
|---|---|---|---|
|  | nt 2.400 ± 600 | nt 7.730 ± 100 | nt 17.850 ± 100 |

Generell werden unter einem Orf-Virus (ORFV) sämtliche unter die Art *Parapoxvirus ovis* fallenden Viren und Virenstämme verstanden. Erfindungsgemäß beruht der anspruchsgemäße rekombinante Vektor auf dem Stamm D1701.

Erfindungsgemäß wird unter einem rekombinanten ORFV-Vektor ein auf dem ORFV-Genom basierender Vektor verstanden, der zum Transport und/oder zur Expression eines Fremdgens in biologische(n) Zellen ausgebildet ist.

Erfindungsgemäß wird unter einem Fremdgen ein nicht dem ORFV-Genom entstammendes Gen bzw. offenes Leseraster (OLR) verstanden.

Unter einem Promotor wird erfindungsgemäß ein solcher Nukleinsäureabschnitt verstanden, der die regulierte Expression des Fremdgens in dem erfindungsgemäßen ORFV-Vektor ermöglicht. Vorzugsweise handelt es sich um einen ORF-Promotor, d.h. um einen im Wildtyp-ORFV-Genom vorliegenden Promotor oder einen sich hiervon ableitenden und ggf. artifiziellen Promotor, wie einen Poxvirus-Promotor, CMV-Promotor etc.

Bei dem rekombinanten ORFV-Vektor lässt sich die Lage der erfindungsgemäßen Insertionsloci IL 2 und 3 im ORFV-Genom erfindungsgemäß auf verschiedene Weisen festlegen: anhand von Restriktionsfragmenten, der ORFV-Gene bzw. offenen Leseraster (OLR) oder Nukleotidpositionen im ORFV-Genom.

Die traditionelle Beschreibung der Lokalisierung von IL 1 (nicht beansprucht), 2 und 3 erfolgt anhand von Restriktionskarten und der Angabe von Restriktionsfragmenten, auf denen die Insertionsbereiche liegen. Die Restriktionskarte des ORFV ist beispielhaft für den Stamm D1701 in Cottone et al. (1998), Analysis of genomic rearrangement and subsequent gene deletion of the attenuated Orf virus strain D1701, Virus Research, Vol. 56, Seiten 53-67, angegeben. Dabei bedeutet erfindungsgemäß bspw. für IL 1 die Angabe Hind*III*-Fragment C, Kpn/ Fragment G, Bam*HI*-Fragment C/G, Eco*RI*-Fragment B, dass sich dieser Insertionslokus von dem Hind*III*-Fragment C bis hin zum Eco*RI*-Fragment B erstreckt. IL 2 erstreckt sich von dem Hind*III*-Fragment I-J bis hin zum Eco*RI*-Fragment A/E. IL 3 erstreckt sich von dem Hind*III*-Fragment G/D bis zum Eco*RI*-Fragment D.

Die Angabe 006, 007 (dUTPase), 008 (G1L-Ank), 009 (G2L) für IL 1 bedeutet, dass sich der Insertionslokus vom Gen bzw. OLR 006 bis hin zum Gen bzw. OLR 009 erstreckt. Die Angaben in den Klammern beziehen sich auf die Codierungsprodukte bzw. codierten enzymatischen Aktivitäten, soweit sie derzeit bekannt sind.

IL 1 liegt erfindungsgemäß in einem Bereich, der bei Nukleotid 400 bis 600 (500 ± 100) beginnt und bei Nukleotid 1800 bis 3000 (2.400 ± 600) endet.

Das für IL 1 Gesagte gilt für IL 2 und IL 3 entsprechend der Angaben in der vorstehenden Tabelle.

Die angegebenen Positionen der Nukleotide (nt) konnten die Erfinder aus einer Vielzahl von Bestimmungen an verschiedenen ORFV-Stämmen ermitteln. Dabei ergaben die Untersuchungen für den Stamm D1701 bzw. Varianten hiervon folgende Positionen:

IL 1:   nt 496 – nt 2.750; nt 496 – nt 1.912 und nt 511 – 2.750

IL 2:   nt 5.210 – 7.736

IL 3:   nt 15.656 – 17.849.

Die Erfinder konnten feststellen, dass in die angegebenen neu erkannten Insertionsloci Fremdgene stabil in das ORFV-Genom integriert werden können. Dies war überraschend. Bislang wurde angenommen, dass die von den Insertionslokussen betroffenen Bereiche im Genom für die Virusreplikation erforderlich bzw. für die Expression von Fremdgenen ungeeignet sind.

Dabei hat sich als besonderer Vorteil des erfindungsgemäßen ORFV-Vektors herausgestellt, dass über die Wahl des Insertionslokus die Genexpression gesteuert werden kann. So ist beispielsweise die Expression von Fremdgenen in IL 2 gegenüber dem bisher verwendeten VEGF-Lokus um den Faktor 2 erniedrigt. Ferner lässt sich über die Wahl des Promotors die Stärke und der Zeitpunkt der Expression des Fremdgens gezielt beeinflussen.

Die der Erfindung zugrundeliegende Aufgabe wird hiermit vollkommen gelöst.

Nach der erfindungsgemäßen Ausgestaltung des rekombinanten ORFV-Vektors handelt es sich bei ORFV um ein solches des Stammes D1701.

Diese Maßnahme hat den Vorteil, dass ein solcher Vektor zum Einsatz kommt, der attenuiert ist und bei einem Wirt nur asymptomatische Infektionen hervorruft. Offenbart hiermit sind sämtliche Varianten von D1701, einschließlich D1701-B und D1701-V.

Nach einer weiteren erfindungsgemäß bevorzugten Ausgestaltung des rekombinanten ORFV-Vektors ist der Promotor ein ORFV-Promotor, weiter vorzugsweise ein früher ORFV-Promotor, der weiter vorzugsweise eine Nukleotidsequenz aufweist, die ausgewählt ist: SEQ ID Nr. 1 (P1 Promotor), SEQ ID Nr. 2 (P2 Promotor), SEQ ID Nr. 3 (optimierter "early" Promotor), SEQ ID Nr. 4 (7.5 kDa Promotor), SEQ ID Nr. 5 (VEGF Promotor) und SEQ ID Nr. 6 (Consensus "early" Promotor).

Diese Maßnahme hat den Vorteil, dass solche Promotoren zum Einsatz kommen, die ein hohes Expressionsniveau des Fremdgens und gezielte Steuerung der Expression ermöglichen. Dabei handelt es sich bei den Promotoren P1 und P2 um solche, die von den Erfindern neu entwickelt wurden. Die übrigen Promotoren stammen vom Vaccinia-Virus und sind in anderen Zusammenhängen in Davidson und Moss (1989), Structure of vaccinia virus late promotors, J. Mol. Biol., Vol. 210, Seiten 771 bis 784, und Yang et al. (2011), Genome-wide analysis of the 5' and 3' ends of Vaccinia Virus early mRNAs delineates regulatory sequences of annotated and anomalous transcripts, J. Virology, Vol. 85, Nr. 12, S. 5897-5909, Broyles (2003), Vaccinia virus transcription, J. Gen. Virol., Vol. 84, Nr. 9, S. 2293-2303, beschrieben. Nach den Erkenntnissen der Erfinder bewirkt P2 eine deutlich höhere Expressionsstärke als P1. Dies war überraschend, da der Promotor P1 zu 100% der Consensussequenz aus dem Vaccinia Virus entspricht, nicht aber P2. Die niedrige Expression des "optimalen" Vaccinia Virus-Promotor (Orthopox) im ORFV (Parapox) ist ein Widerspruch und überraschend. Zudem überrascht, dass der P2-Promotor zu sehr starke Expression führt.

Nach einer weiteren bevorzugten Ausgestaltung des erfindungsgemäßen rekombinanten ORFV-Vektors ist der Promotor an einer Position von nt 28 $\pm$ 10 bis nt - 13 $\pm$ 10 stromaufwärts in Bezug auf die für das Fremdgen codierende Nukleotidsequenz angeordnet.

Diese Maßnahme hat den Vorteil, dass der Promotor an einer solchen Position lokalisiert ist, die eine hohe Expressionsstärke und kontrollierte Expression des Fremdgens ermöglicht.

Nach einer weiteren bevorzugten Ausgestaltung des erfindungsgemäßen rekombinanten ORFV-Vektors ist in zumindest einem der IL 1, 2 oder 3 mehr als eine, vorzugsweise 2, 3, 4 oder mehr, für ein Fremdgen codierende und dieses exprimierende Nukleotidsequenz eingesetzt.

Diese Maßnahme hat den Vorteil, dass mit einem rekombinanten erfindungsgemäßen ORFV-Vektor mehrere Fremdgene exprimiert werden können. Diese Ausgestaltung eignet sich besonders für die Herstellung von polyvalenten Impfstoffen, die zugleich gegen mehrere antigene Strukturen gerichtet sind. Dabei können in jedem Insertionslokus mehrere Fremdgene, vorzugsweise 2, 3, 4 oder mehr, exprimiert werden.

Nach einer erfindungsgemäß bevorzugten Ausgestaltung weist der rekombinante ORFV-Vektor eine weitere für ein Fremdgen codierende und dieses exprimierende Nukleotidsequenz auf, die unter der Kontrolle eines vorzugsweise frühen ORFV-Promotors steht, und die einen Insertionslokus eingesetzt ist, der im ORFV-Genom im vegf-E-Gen lokalisiert ist.

Diese Maßnahme hat den Vorteil, dass ein im Stand der Technik bereits beschriebener und gut charakterisierter Insertionslokus zum Einsatz kommt. Die Verwendung des vegf-Lokus kann für eine gezielte Steuerung der Genexpression genutzt werden. So kann im vegf-Lokus die Expression des Fremdgens im Vergleich zu einem der neuen Expressionslokusse, z.B. IL 2, erhöht sein.

Nach einer erfindungsgemäß bevorzugten Ausgestaltung ist das Fremdgen des rekombinanten ORFV-Vektors ausgewählt aus den Gruppen folgender Antigene:

- virales Antigen, vorzugsweise

     Rabiesvirus-Antigen, einschließlich Glykoprotein (RabG);

     Influenza-A-Antigen, einschließlich Nukleoprotein (NP), Hämaglutinin (HA), Neuraminidase (NA);

- Tumorantigen, vorzugsweise virales Tumorantigen, einschließlich HPV-selektives virales Tumorantigen;

- tumorassoziiertes Antigen, einschließlich virales tumorassoziiertes Antigen, einschließlich HPV-selektives virales tumorassoziiertes Antigen;

- parasitäres Antigen, vorzugsweise Plasmodium-Antigen;

- Zytokin.

Diese Maßnahme hat den Vorteil, dass über das erfindungsgemäße rekombinante ORFV-Virus besonders bedeutsame Antigene, insbesondere für die Herstellung von Impfstoffen, exprimierbar sind.

Ein weiterer Gegenstand der vorliegenden Erfindung betrifft eine biologische Zelle, vorzugsweise eine Säugetierzelle, weiter vorzugsweise eine Vero-Zelle, enthaltend den erfindungsgemäßen ORFV-Vektor.

Ein weiterer Gegenstand der vorliegenden Erfindung betrifft eine Zusammensetzung, vorzugsweise eine pharmazeutische Zusammensetzung, enthaltend den erfindungsgemäßen ORFV-Vektor oder/und die erfindungsgemäße Zelle. Bei der pharmazeutischen Zusammensetzung kann es sich vorzugsweise um einen Impfstoff, weiter vorzugsweise um einen polyvalenten Impfstoff handeln.

Die Eigenschaften, Vorteile, Merkmale und Weiterbildungen des erfindungsgemäßen rekombinanten ORFV-Vektors gelten für die erfindungsgemäße Zelle und die erfindungsgemäße Zusammensetzung entsprechend.

Ein weiterer Gegenstand der vorliegenden Erfindung betrifft die Verwendung des erfindungsgemäßen rekombinanten ORFV-Vektors zur Expression von zumindest einem Fremdgen, weiter vorzugsweise zur Expression von zumindest einem ein Fremdgenprodukt enthaltenden Impfstoff (monovalenter Impfstoff), weiter vorzugsweise von einem zumindest zwei Fremdgenprodukte enthaltenden Impfstoff (polyvalenter Impfstoff).

In diesem Zusammenhang bedeutet "zumindest", dass 2, 3, 4, 5, 6, 7, 8, 9 etc. Fremdgene umfasst sind.

Die Merkmale, Vorteile, Eigenschaften und Weiterbildungen des erfindungsgemäßen rekombinanten ORFV-Vektors gelten für die erfindungsgemäße Verwendung entsprechend.

Ein weiterer Gegenstand der vorliegenden Erfindung betrifft ein Nukleinsäuremolekül codierend für einen ORFV-Promotor, vorzugsweise einen frühen ORFV-Promotor, der eine Nukleotidsequenz aufweist, die ausgewählt ist aus den Nukleotidsequenzen SEQ-ID Nr. 1 (P1) und SEQ-ID Nr. 2 (P2).

Das erfindungsgemäße Nukleinsäuremolekül codiert für neue ORFV-Promotoren, die für die Expression von Fremdgenen in dem erfindungsgemäßen rekombinanten ORFV-Vektor besonders geeignet sind. Die Promotoren bewirken eine sehr starke, frühe Genexpression.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind.

Die Erfindung wird nun anhand von Ausführungsbeispielen näher erläutert, aus denen sich weitere Merkmale, Vorteile und Eigenschaften der Erfindung ergeben. Dabei wird Bezug genommen auf beiliegende Figuren, in denen Folgendes dargestellt ist:

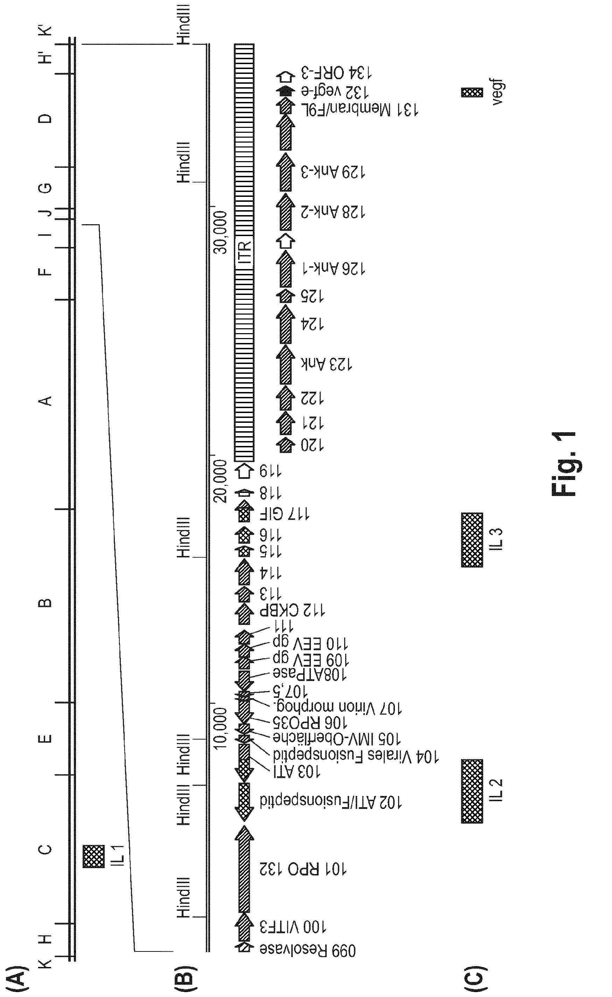

Fig. 1 zeigt die Karte der Hind *III*-Restriktionsfragmente des ORFV D1701-V DNA-Genoms. Die schraffierten Kästen stellen die Insertionsstellen IL1, IL2, IL3 und vegf dar. ITRL steht für die invertierten terminalen Repeats der Genomenden.

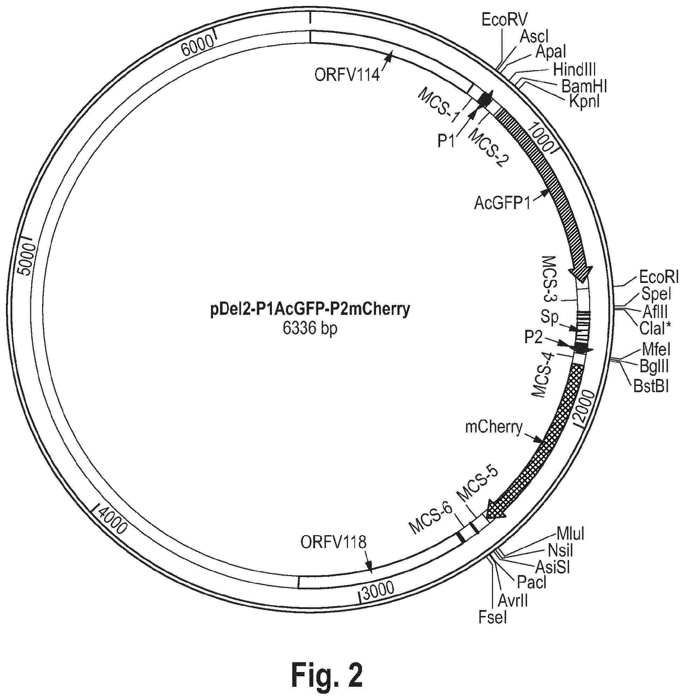

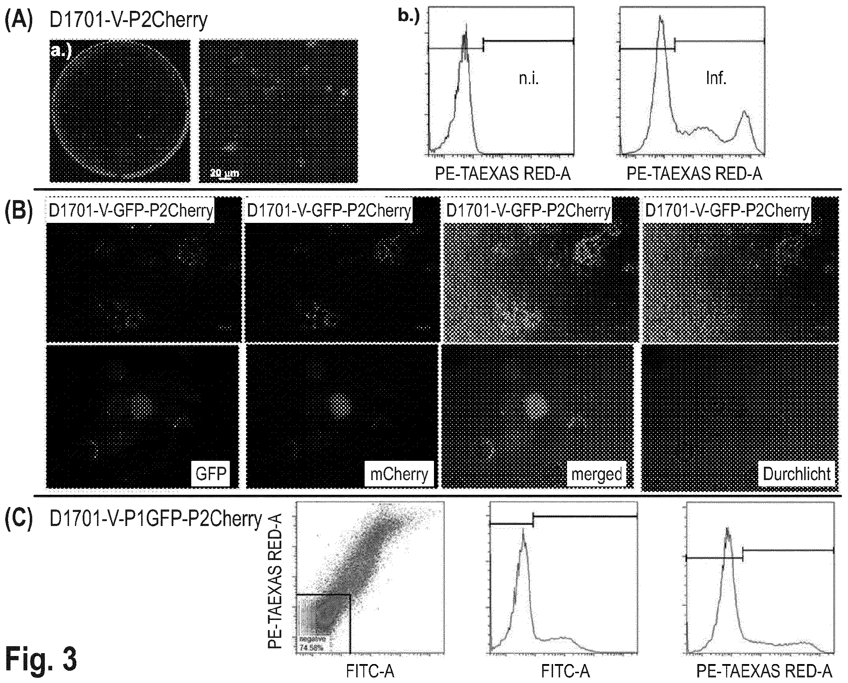

Fig. 2 zeigt eine schematische Darstellung des Transferplasmids pD1-GFP-D2Cherry. Der Vektor zeigt das AcGFP-Gen (Linienschraffur), das unter der Kontrolle des artifiziellen frühen Promotors P1 (schwarzer Pfeil, oben) steht, sowie das mCherry-Gen (Kästchenschraffur), das unter der Kontrolle des artifiziellen frühen Promotors P2 (schwarzer Pfeil, rechts) steht. Hinter beiden Fluoreszenzgenen befinden sich poxvirusspezifische frühe Transkriptionsstopp-Motive T5NT (schwarz). Die Gene sind über einen Spacer (Sp) voneinander getrennt. Mehrere multipler Klonierungsstellen (MCS 1-6) ermöglichen den Austausch der Fluoreszenzmarkergene durch gewünschte Fremdgene. Die Gene umfassenden flankierten Bereiche sind stromabwärts homolog zu dem ORFV-Genombereich ORF117/118, stromaufwärts homolog zu dem ORFV-Genombereich ORF114, und gewährleisten eine zielgerichtete Integration in den IL 3-Lokus des D1701-V-Genoms über homologe Rekombination.

Fig. 3 zeigt eine Expressionsanalyse unterschiedlicher Fluoreszenzrekombinanten. (A,a) Fluoreszenz-mikroskopische Aufnahme einer 6-Well-Platte mit D1701-V-D2Cherry infizierten Vero-Zellen. Zur Selektion der Rekombinanten wurden Cherry-fluoreszierende Plaques gepickt und das Virus aus den Plaques angezogen. Nach vier Plaque-Reinigungen konnte die Homogenität von D1701-V-D2Cherry über PCR-Analysen sichergestellt werden. (A,b) Bestimmung der Cherry-Expression mittels Durchflußzytometrie. Die Abbildung zeigt exemplarisch die Expression D1701-V-D2Cherry infizierter Vero-Zellen (MOI = 1,0) im Durchflußzytometer. Nach 48 Stunden exprimieren ca. 45 % aller lebender Einzelzellen Cherry. Nicht-infizierte Vero-Zellen dienten als Negativkontrolle. (B) Fluoreszenzexpression der Rekombinante D1701-V-GFP-D2Cherry. Vero-Zellen wurden mit D1701-V-GFP-D2Cherry infiziert (MOI = 0,5). In der oberen Reihe ist eine Fluoreszenzaufnahme 48 Stunden nach Infektion zu sehen (Vergrößerung: 20X). Die untere Reihe zeigt die Fluoreszenzexpression nach 24 Stunden (Vergrößerung: 63X). Die Fluoreszenzmikroskopie erlaubte die Darstellung der AcGFP-(GFP), der mCherry-Expression (mCherry), sowie beider Fluoreszenzen in einer Zelle (merged). Zusätzlich wurden die Zellen im Mikroskop-Durchlicht (Durchlicht) aufgenommen. (C) Fluoreszenzexpression der Rekombinante D1701-V-D1GFP-D2Cherry. Vero-Zellen wurden mit D1701-V-D1GFP-P2Cherry infiziert (MOI = 1,0) und die Expression im Durchflußzytometer ermittelt. Nach 24 Stunden exprimierten ca. 25 % aller lebenden Einzelzellen sowohl mCherry, als auch GFP. Nicht-infizierte Vero-Zellen dienten als Negativkontrolle.

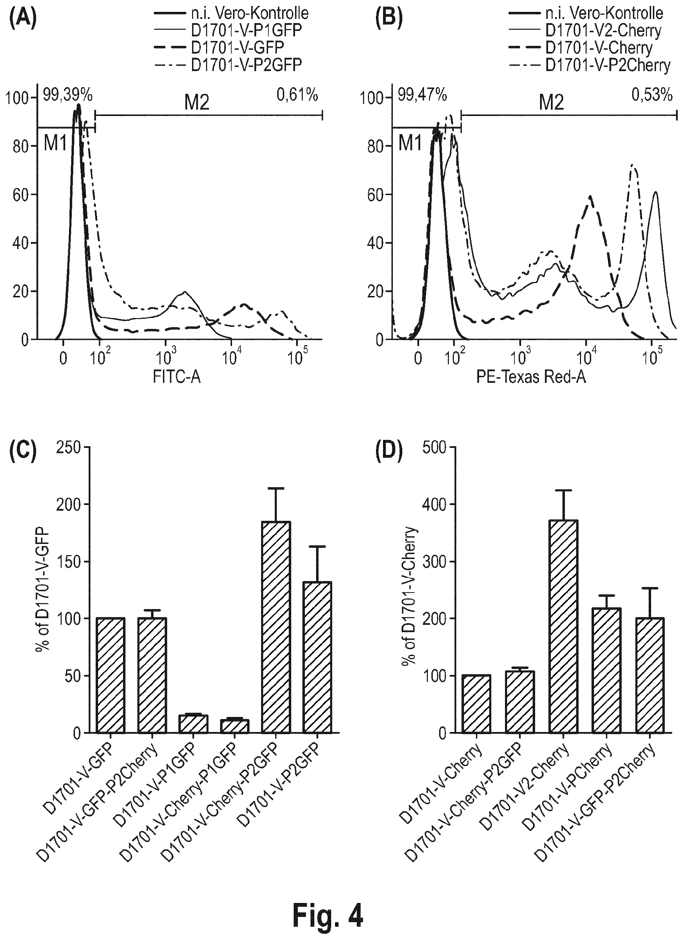

Fig. 4 zeigt die Bestimmung der Fluoreszenzintensität unterschiedlicher Rekombinanten. (A) Vero-Zellen wurden mit GFP-exprimierenden Rekombinanten (MOI ca. 1,5) infiziert und 24 Stunden später die mittlere Fluoreszenzintensität durchflußzytometrisch bestimmt. Nicht-infizierte Vero-Zellen dienten als Negativkontrolle. M1 beschreibt den Bereich in dem 99,39 % aller nicht-infizierten Zellen (vordere erste Kurve) detektiert werden. Im Bereich M2 befinden sich dagegen GFP-positive Zellen. Die Population GFP-positiver Zellen war nach Infektion mit den GFP-exprimierenden Rekombinanten vergleichbar (38,2 % - 40,0 %). Dabei war ersichtlich, dass die GFP-Intensität in D1701-V-D1GFP-infizierten Zellen (durchgezogene Linie) am geringsten, in D1701-V-D2GFP-infizierten Zellen (----) am stärksten war. (B) Vero-Zellen wurden mit mCherryexprimierenden Rekombinanten (MOI ca. 3,0) infiziert und 24 Stunden später die mittlere Fluoreszenzintensität durchflußzytometrisch bestimmt. Nicht-infizierte Vero-Zellen dienten als Negativkontrolle. M1 beschreibt den Bereich in dem 99,47 % aller nicht-infizierten Zellen (vordere erste Kurve) detektiert werden. Im Bereich M2 befinden sich dagegen Cherry-positive Zellen. Die Population mCherry-positiver Zellen war nach Infektion mit den GFP-exprimierenden Rekombinanten vergleichbar (62,5 % - 63,3 %). Dabei war die mCherry-Intensität in D1701-V-Cherry-infizierte Zellen (- - - - -) deutlich geringer als in D1701-V-D2Cherry- (blaue Linie) bzw. in D1701-V2Cherry-infizierten Zellen (rote Linie). (C+D) Die Diagramme zeigen die prozentuale Fluoreszenzintensität unterschiedlicher Fluoreszenz-rekombinanten in Relation zu D1701-V-GFP (C) bzw. zu D1701-V-Cherry (D). Die Daten repräsentieren Mittelwerte aus mindestens 3 unabhängigen Experimenten.

Ausführungsbeispiele

## 1. Das ORFV-Genom

Das ORFV-Genom besteht aus einer linearen Doppelstrang-DNA und hat eine Länge von etwa 138 kB, einen GC-Gehalt von etwa 64 % und besitzt 130-132 Gene. Der Aufbau des ORFV-Genoms ähnelt dem anderer Poxviren. Es besteht aus einer zentralen Region mit essentiellen Genen, die einen hohen Grad an Konservierung innerhalb der Poxviridae aufweisen. So finden sich im ORFV-Genom 88 Gene, die bei allen Chordopoxvirinae konserviert auftreten. In den terminalen Regionen sind virale Gene lokalisiert, die für das *in vitro* Wachstum nicht essentiell, jedoch für die Pathogenität und den Tropismus des Virus relevant sind.

Im Vergleich zu anderen Orf Viren zeigt das an die Vermehrung in Zellkultur angepasste D1701 Virus eine deutliche Vergrößerung der invertierten terminalen Repeats (ITR). Diese Veränderungen führten nicht nur zum Verlust, sondern auch zur Duplizierung einiger Gene, wie auch dem vegf-e-Gen. Die Adaptierung des in bovinen BK-KL3A Zellen vermehrten D1701-B an das Wachstum in Vero Zellen erzeugte drei weitere Insertionslokusse IL 1, IL 2 und IL 3 im Virusgenom des nun D1701-V bezeichneten Virus. Diese sind in der Fig. 1 veranschaulicht.

## 2. Poxvirus-Promotoren

Poxviren besitzen "early", "intermediate" und "late" Promotoren. Diese unterschiedlichen Promotoren weisen einige charakteristische Sequenzeigenschaften auf, die am Beispiel des VACV im Folgenden erläutert werden. So besteht der "early" Promotor des VACV aus einer 16 bzw. 15 Nukleotide langen kritischen Region, die von einer 7 Nukleotide langen Initiatorregion durch eine 11 Nukleotide lange Abstandsregion getrennt ist. Die kritische Region ist eher adeninreich, wohingegen die Abstandsregion eher thyminreich ist. Die Initiation der Transkription erfolgt mit seltenen Ausnahmen stets an einem Purin. Nukleotid-Substitutionen in der kritischen Region können einen drastisch negativen Effekt auf die Promotoraktivität haben, selbst ein vollständiger Verlust der Aktivität ist möglich. Substitutionsanalysen des frühen VACV 7.5 kDa Promotors lieferten eine optimierte kritische Region, zudem ist es gelungen, eine Consensus-Sequenz für den "early" Poxviruspromotor abzuleiten, die in der Tabelle 1 gezeigt ist. Die "intermediate" Promotoren bestehen aus einer AT-reichen Kernsequenz, die etwa 14 Nukleotide lang ist, gefolgt von einer 10-11 Nukleotide langen Abstandsregion, woran sich eine kurze Initiatorregion anschließt. Der Aufbau der "late" Promotoren besteht aus einer vorgelagerten etwa 20 Nukleotide langen AT-reichen Region, die durch eine Abstandsregion vor der Transkriptionsstartstelle von etwa 6 Nukleotide getrennt ist, welche die hochkonservierte Sequenz -1 TAAAT +4 beinhaltet.

| | kritische Region | | | | | | | | | | | | | | | | SEQ ID | % Überein-stimmung mit opt. "early" | Abstandsregion Thymin reich -12 bis -2 | Initiator Region Start meist an Purin -1 bis 6 |
|---|---|---|---|---|---|---|---|---|---|---|---|---|---|---|---|---|---|---|---|---|
| | -28 | -27 | -26 | -25 | -24 | -23 | -22 | -21 | -20 | -19 | -18 | -17 | -16 | -15 | -14 | -13 | | | | |
| optimierter "early" | A | A | A | A | A | T | T | G | A | A | A | A | A | C/T | T | A | 3 | | | |
| 7.5 kDa Promotor | A | A | A | A | G | T | A | G | A | A | A | A | T | | T | A | 4 | 75 | | |
| Consesus "early" | A | A | A | A | A | A | T | G | A | A | A | A | A | A | A/T | A | 6 | 87,5 | | |
| VEGF | C | A | Â | A | A | T | G | T | A | A | A | T | T | A | T | A | 5 | 62,5 | | |
| P1 | A | A | A | A | A | T | T | G | A | A | A | A | A | T | T | A | 1 | 100 | | |
| P2 | A | A | A | A | A | T | T | G | A | A | A | T | T | C | T | A | 2 | 87,5 | | |

Tab. 1: Eingesetzte Promotoren; P1 und P2 wurden von den Erfindern neu entwickelt.

3. Herstellung des rekombinanten ORFV-Vektors

Die Erfinder haben nach einer neuen Strategie zur Herstellung eines rekombinanten polyvalenten ORFV-Vektors gesucht. Während der Adaption des ORFV auf Vero-Kulturzellen sind mehreren Deletionen im viralen Genom aufgetreten. Es wurde untersucht, ob sich die Deletionsbereiche für die Integration von Fremdgenen eignen (Fig. 1A).

Daher wurde neben weiteren Plasmiden das Transferplasmid pDel2 konzipiert, welches die homologen Bereiche der IL 3-Region umfasst (Fig. 2). Die Klonierung von Fremdgenen in das Plasmid wurde durch den Einsatz mehrerer MCS (multipler Klonierungsstellen) ermöglicht. Zusätzlich wurde das Plasmid so konstruiert, dass es die gleichzeitige Integration mehrerer Fremdgene erlaubte, die jeweils unter Kontrolle artifizieller früher ORFV-Promotoren stehen und von poxvirusspezifischen T5NT frühen Transkriptions-Stopp-Motiven begrenzt sind (Fig. 2).

Es wurden Nukleotidsequenzen der neuen artifiziellen frühen ORFV-Promotoren P1 und P2 entworfen.

In einem ersten Versuch sollte untersucht werden, ob der IL 3-Lokus für die stabile Integration von Fremdgenen geeignet ist. Hierzu wurde das mCherry-Fluoreszenzmarkergen unter Kontrolle des Promoters P2 in das pDel2-Transferplasmid kloniert. Anschließend wurde das Plasmid in mit D1701-VrV-infizierte Vero-Zellen transfiziert und neue rekombinante Viren visuell nach Identifikation rot-leuchtender Zellen mittels Fluoreszenzmikroskopie selektiert und die über mehrere Plaquereinigungen erhaltene homogene Rekombinante D1701-V-D2-Cherry angezogen (Fig. 3A,a).

Die korrekte Integration des mCherry-Gens in den IL 3-Lokus von D1701-VrV wurde über spezifische PCR-Analysen und Southern Blot-Hybridisierungen sichergestellt, die korrekte Expression ließ sich neben Fluoreszenz-und Western Blot-Analysen auch mittels Durchflußzytometrie zeigen (Fig. 3A,b).

Dabei konnte eine starke Expression früh nach der Infektion nachgewiesen werden. Durch mehrmaliges *in vitro* Passagieren der Rekombinante konnte gezeigt werden, dass die Fremdgen-Integration in das ORFV-Genom stabil war.

Weiterhin wurde mithilfe der hergestellten Rekombinante D1701-V-GFP-D2-Cherry, bei der das AcGFP-Gen im vegf-e- und das mCherry-Gen im IL 3-Lokus integriert ist, erfolgreich die simultane frühe Expression zweier Fluoreszenzgene in unterschiedlichen Insertionslokussen nachgewiesen (Fig. 3B).

Weiter sollte untersucht werden, ob gleichzeitig auch ein zweites Fremdgen stabil in den IL 3-Lokus integriert werden kann. Hierfür wurde in das pDel2-Transferplasmid neben dem P2-kontrollierten mCherry-Gen, das AcGFP-Gen unter Kontrolle des P1-Promoters kloniert. Die Selektion und Aufreinigung der homologen Rekombinanten D1701-V-D1-GFP-D2-Cherry erfolgte analog zu der vorher beschriebenen D1701-V-P2-Cherry-Selektion.

Auch hier wurde über PCR- und Southern Blot Analysen die korrekte Integration der beiden Fremdgene in den IL 3-Lokus gezeigt. Der Nachweis der Expression erfolgte über Fluoreszenzmikroskopie und Durchflußzytometrie (Fig. 3C).

Die Stärke der Promotoren P1 und P2 wurde untereinander und mit dem Promoter $P_{vegf}$ in Expressionsanalysen verglichen. Dabei zeigte sich, dass der Promoter P2 die stärkste, der Promoter P1 die schwächste Genexpression induzierte (Fig. 4A + 4C). Dies war überraschend, da P1 zu 100% der Consenssequenz aus dem Vaccinia Virus entspricht, nicht aber P2.

Zudem konnte nachgewiesen werden, dass die Integration eines zweiten, unter Kontrolle eines eigenständigen Promoters regulierten Fremdgens keine Auswirkung auf die Expressionsstärke des ersten Fremdgens hatte. Dabei war es irrelevant, ob das zweite Gen in denselben oder in einen unterschiedlichen Insertionslokus integriert wurde. Nach der Insertion eines P2-gesteuerten mCherry-Gens in den VEGF-Lokus konnte anhand des Vergleichs mit der Rekombinanten, die das P2-regulierte mCherry-Gen in den IL 3-Lokus integriert hatte, der Einfluss des Insertionsorts untersucht werden. Dabei zeigte sich, dass die Genexpression im VEGF-Lokus etwa zweimal so stark war, wie im IL 3-Lokus (Fig. 4B + 4D).

Zusammenfassend konnte gezeigt werden, dass der Orf-Virusvektor D1701-V für die Herstellung polyvalenter Rekombinanten sehr gut geeignet ist. Es konnten mehrere Fremdgene stabil in das virale Genom integriert werden, bspw. über die neu aufgefundenen Insertionslokusse IL 1, 2 und 3, oder aber den bekannten Insertionslokus VEGF. Die Stärke der Fremdgenexpression ist sowohl von dem Promotor als auch vom Insertionslokus abhängig. Die stärkste Genexpression wurde nach Integration eines P2-gesteuerten Fremdgens im VEGF-Lokus erreicht.

Von den Erfindern wurden weitere unterschiedliche Vektoren hergestellt, die sich durch die Art und Konstellation der verschiedenen Marker-Fremdgene, Insertionsorte und Promotoren voneinander unterscheiden (Tab. 2).

Tab. 2: Tabellarische Übersicht der neu hergestellten fluoreszierenden ORFV-Vektoren.

| Rekombinante | Lokus | | | Fremdgenexpression |
|---|---|---|---|---|
| | VEGF | IL3 | | |
| D1701-V-Cherry | $P_{vegf}$: mCherry | - | - | +++ |
| D1701-V-Cherry-D1GFP | $P_{vegf}$: mCherry | P1:AcGFP | - | +++/+ |
| D1701-V-Cherry-D2GFP | $P_{vegf}$: mCherry | - | P2: AcGFP | +++/++++ |
| D1701-V12-Cherry | P2: mCherry | - | - | ++++ |
| D1701-V12-Cherry-D2GFP | P2: mCherry | - | P2: AcGFP | ++++/++++ |
| D1701-V-GFP | $P_{vegf}$: AcGFP | - | - | +++ |
| D1701-V-GFP-D2Cherry | $P_{vegf}$: AcGFP | - | P2: mCherry | +++/++++ |
| D1701-V-GFP-D2CD4 | $P_{vegf}$: AcGFP | - | P2: hCD4 | +++/++++ |
| D1701-V-D1GFP | $P_{vegf}$: LacZ | P1: AcGFP | - | +++/+ |
| D1701-V-D1GFP-D2Cherry | $P_{vegf}$: LacZ | P1: AcGFP | P2: mCherry | +++/+/++++ |
| D1701-V-D2GFP | $P_{vegf}$: LacZ | - | P2: AcGFP | +++/++++ |
| D1701-V-D2Cherry | $P_{vegf}$: LacZ | - | P2: mCherry | +++/++++ |
| D1701-V-D2Orange | $P_{vegf}$: LacZ | - | P2: mOrange | +++/++++ |
| D1701-V-CD4-D2Cherry | $P_{vegf}$: hCD4 | - | P2: mCherry | +++/++++ |

Die Tabelle gibt einen Überblick über die während der zur Erfindung führenden Arbeiten hergestellten fluoreszierenden rekombinanten ORFV-Vektoren. Der Insertionsort (Lokus) und die für die Steuerung der Fremdgenexpression genutzten Promotoren (P$_{vegf}$, P1, P2) sind für die jeweiligen Rekombinanten in der Tabelle ersichtlich. Zudem ist die Stärke der Fremdgenexpression angegeben (sehr stark = ++++ bis schwach = +).

Die Erfindung eröffnet für die Entwicklung neuer rekombinanter ORFV-basierter Impfstoffe eine Vielzahl an Optionen. So können Rekombinanten hergestellt werden, die gleichzeitig mehrere Antigene exprimieren. Dies könnte etwa bei der Herstellung einer universellen Vakzine, von Kombinationsimpfstoffen oder von therapeutischen Tumorimpfstoffen, die sich gegen mehrere Tumorantigene richten, ein bedeutender Vorteil sein. Darüber hinaus könnte die Immunantwort durch eine gleichzeitige Insertion von Antigen und Zytokinen gezielt beeinflusst werden.

**Patentansprüche**

1. Rekombinanter Orf-Virus-(ORFV)-Vektor, der folgendes aufweist:

   (1) zumindest eine für ein Fremdgen codierende und dieses exprimierende Nukleotidsequenz, und
   (2) zumindest einen Promotor, der die Expression der Nukleotidsequenz kontrolliert,

   **dadurch gekennzeichnet, dass**

   die Nukleotidsequenz in zumindest einen der Insertionsloci (IL) 2 und 3 eingesetzt ist, die im ORFV-Genom in folgenden Regionen lokalisiert sind:

   |         | IL 2 | IL 3 |
   |---------|------|------|
   | Gen/OLR | 102, | 114, |
   |         | 103  | 115, |
   |         |      | 116, |
   |         |      | 117 (GIF) |

   **dadurch gekennzeichnet, dass** es sich bei ORFV um ein solches des Stammes D1701 handelt.

2. Rekombinanter ORFV-Vektor nach Anspruch 1, **dadurch gekennzeichnet, dass** der ORFV-Promotor ein früher ORF-Promotor ist, der vorzugsweise eine Nukleotidsequenz aufweist, die ausgewählt ist aus: SEQ ID Nr. 1 (P1 Promotor), SEQ ID Nr. 2 (P2 Promotor), SEQ ID Nr. 3 (optimierter "early" Promotor), SEQ ID Nr. 4 (7.5 kDa Promotor), SEQ ID Nr. 5 (VEGF Promotor) und SEQ ID Nr. 6 (Consensus "early" Promotor).

3. Rekombinanter ORFV-Vektor nach einem der vorherigen Ansprüche, **dadurch gekennzeichnet, dass** der Promotor an einer Position von nt -28 ± 10 bis nt -13 ± 10 stromaufwärts in Bezug auf die für das Fremdgen codierende Nukleotidsequenz angeordnet ist.

4. Rekombinanter ORFV-Vektor nach einem der vorherigen Ansprüche, **dadurch gekennzeichnet, dass** in zumindest einem der IL 2 oder 3 mehr als eine für ein Fremdgen codierende und dieses exprimierende Nukleotidsequenz eingesetzt ist, vorzugsweise 2, 3, 4 oder mehr Nukleotidsequenzen.

5. Rekombinanter ORFV-Vektor nach einem der vorherigen Ansprüche, **dadurch gekennzeichnet, dass** der rekombinante ORFV-Vektor eine weitere für ein Fremdgen codierende und dieses exprimierende Nukleotidsequenz aufweist, die unter der Kontrolle eines vorzugsweise frühen ORFV-Promotors steht und die in einen Insertionslokus eingesetzt ist, der im ORFV-Genom im vegf-E-Gen lokalisiert ist.

6. Rekombinanter ORFV-Vektor nach einem der vorherigen Ansprüche, **dadurch gekennzeichnet, dass** das Fremdgen ausgewählt ist aus den Gruppen folgender Antigene:

   - virales Antigen, vorzugsweise

     Rabiesvirus-Antigen, einschließlich Glykoprotein (RabG);
     Influenza-A-Antigen, einschließlich Nukleoprotein (NP), Hämaglutinin (HA), Neuraminidase (NA);

   - Tumorantigen, vorzugsweise virales Tumorantigen, einschließlich HPV-selektives virales Tumorantigen;

- tumorassoziiertes Antigen, einschließlich virales tumorassoziiertes Antigen, einschließlich HPV-selektives virales tumorassoziiertes Antigen;
- parasitäres Antigen, vorzugsweise Plasmodium-Antigen;
- Zytokin.

**7.** Zelle, vorzugsweise Säugetierzelle, weiter vorzugsweise eine Vero-Zelle, enthaltend den rekombinanten ORFV-Vektor nach einem der vorherigen Ansprüche.

**8.** Zusammensetzung, vorzugsweise pharmazeutische Zusammensetzung, enthaltend den rekombinanten ORFV-Vektor nach einem der Ansprüche 1 bis 6 oder/und die Zelle nach Anspruch 7.

**9.** Zusammensetzung nach Anspruch 8, **dadurch gekennzeichnet, dass** es sich bei der pharmazeutischen Zusammensetzung um einen Impfstoff, vorzugsweise eine polyvalenten Impfstoff handelt.

**10.** Verwendung des rekombinanten ORFV-Vektors nach einem der vorherigen Ansprüche 1 bis 6 zur Expression von zumindest einem Fremdgen, ausgenommen zur Expression *in vivo*.

**11.** Verwendung nach Anspruch 10 zur Herstellung von einem zumindest ein Fremdgenprodukt enthaltenden Impfstoff, welcher ein monovalenter Impfstoff ist, vorzugsweise von einem zumindest zwei Fremdgenprodukte enthaltenden Impfstoff, welcher ein polyvalenter Impfstoff ist.


**Claims**

**1.** Recombinant Orf virus (ORFV) vector comprising:

(1) at least one nucleotide sequence encoding and expressing a foreign gene, and
(2) at least one promoter controlling the expression of the nucleotide sequence,

**characterized in that**:

the nucleotide sequence is localized in at least one of insertion loci (IL) 2 and 3, which are localized in the ORFV genome in the regions below:

|  | IL 2 | IL 3 |
|---|---|---|
| Gene/ORF | 102, 103 | 114, 115, 116, 117 (GIF) |

**characterized in that** the ORFV is of the strain D1701.

**2.** Recombinant ORFV vector of claim 1, **characterized in that** the ORFV promoter is an early ORF promoter, preferably comprising a nucleotide sequence selected from the group consisting of SEQ ID NO: 1 (P1 promoter), SEQ ID NO: 2 (P2 promoter), SEQ ID NO: 3 (optimized "early" promoter), SEQ ID NO: 4 (7.5 kDa promoter), SEQ ID NO: 5 (VEGF promoter) and SEQ ID NO: 6 (Consensus "early" promoter).

**3.** Recombinant ORFV vector of any one of the preceding claims, **characterized in that** the promoter is located at a position of nt -28 $\pm$ 10 to nt -13 $\pm$ 10 upstream in relation to the nucleotide sequence encoding the foreign gene.

**4.** Recombinant ORFV vector of any one of the preceding claims, **characterized in that** in at least one of IL 2 or 3 more than one nucleotide sequence encoding and expressing a foreign gene is inserted, preferably 2, 3, 4 or more nucleotide sequences.

**5.** Recombinant ORFV vector of any one of the preceding claims, **characterized in that** the recombinant ORFV vector comprises a further nucleotide sequence encoding and expressing a foreign gene, which is under the control of a, preferably, early ORFV promoter, and which is inserted into an insertion locus, which is located in the vegf-E-gene in the ORFV genome.

6. Recombinant ORFV vector of any one of the preceding claims, **characterized in that** the foreign gene is selected from the groups of the following antigens:

- a viral antigen, preferably

Rabies virus antigen, including glykoprotein (RabG);
Influenza A antigen, including nucleoprotein (NP), hemagglutinin (HA), neuraminidase (NA);

- tumor antigen; preferably viral tumor antigen, including HPV- selective viral tumor antigen;
- tumor associated antigen; including viral tumor associated antigen, including HPV-selective viral tumor associated antigen;
- parasitic antigen; preferably plasmodium antigen;
- cytokine.

7. Cell, preferably a mammalian cell, further preferably a Vero cell, containing the recombinant ORFV vector according to any one of the preceding claims.

8. Composition, preferably pharmaceutical composition, containing the recombinant ORFV vector of any one of claims 1 to 6 and/or the cell of claim 7.

9. Composition of claim 8, **characterized in that** the pharmaceutical composition is a vaccine, preferably a polyvalent vaccine.

10. Use of the recombinant ORFV vector of any one of the preceding claims 1 to 6 for the expression of at least one foreign gene, excluding for the expression *in vivo.*

11. Use of claim 10 for producing a vaccine comprising at least one foreign gene product, which is a monovalent vaccine, preferably of a vaccine comprising at least two foreign gene products, which is a polyvalent vaccine.

**Revendications**

1. Vecteur recombinant du virus de l'Orf (ORFV), qui présente :

(1) au moins une séquence nucléotidique codant pour un gène étranger et exprimant celui-ci et
(2) au moins un promoteur qui contrôle l'expression de la séquence nucléotidique,
**caractérisé en ce que** la séquence nucléotidique est utilisée dans au moins l'un des locus d'insertion (IL) 2 et 3, qui sont localisés dans les régions suivantes dans le génome du ORFV :

|  | IL2 | IL 3 |
|---|---|---|
| Gène/OLR | 102, | 114, |
|  | 103 | 115, |
|  |  | 116, |
|  |  | 117 (GIF) |

**caractérisé en ce qu'**il s'agit, pour l'ORFV, d'un virus de la souche D1701.

2. Vecteur recombinant d'ORFV selon la revendication 1, **caractérisé en ce que** le promoteur de l'ORFV est un promoteur d'ORF précoce qui présente de préférence une séquence nucléotidique choisie parmi : SEQ ID NO 1 (promoteur P1), SEQ ID NO 2 (promoteur P2), SEQ ID NO 3 (promoteur "précoce" optimisé), SEQ ID NO 4 (promoteur de 7,5 kDa), SEQ ID NO 5 (promoteur du VEGF) et SEQ ID NO 6 (promoteur "précoce" de consensus).

**3.** Vecteur recombinant d'ORFV selon l'une des revendications précédentes, **caractérisé en ce que** le promoteur est disposé en une position de nt - 28 ± 10 à nt - 13 ± 10 en amont par rapport à la séquence nucléotidique codant pour le gène étranger.

**4.** Vecteur recombinant d'ORFV selon l'une des revendications précédentes, **caractérisé en ce que** dans au moins l'un des locus IL 2 ou 3, plus d'une séquence nucléotidique codant pour un gène étranger et exprimant celui-ci est utilisée, de préférence 2, 3, 4 séquences nucléotidiques ou plus.

**5.** Vecteur recombinant d'ORFV selon l'une des revendications précédentes, **caractérisé en ce que** le vecteur recombinant d'ORFV présente une autre séquence nucléotidique codant pour un gène étranger et exprimant celui-ci, qui est sous le contrôle d'un promoteur d'ORFV de préférence précoce et qui est utilisée dans un locus d'insertion qui est localisé dans le génome d'ORFV dans le gène vegf-E.

**6.** Vecteur recombinant d'ORFV selon l'une des revendications précédentes, **caractérisé en ce que** le gène étranger est choisi dans les groupes des antigènes suivants :

- un antigène viral, de préférence

un antigène du virus de la rage, y compris une glycoprotéine (RabG) ;
un antigène de l'influenza-A, y compris une nucléoprotéine (NP), une hémagglutinine (HA), une neuraminidase (NA) ;

- un antigène tumoral, de préférence un antigène tumoral viral, y compris un antigène tumoral viral sélectif pour le VPH ;
- un antigène associé à une tumeur, y compris un antigène viral associé à une tumeur, y compris un antigène viral associé à une tumeur sélectif pour le VPH ;
- un antigène parasitaire, de préférence antigène de plasmodium ;
- une cytokine.

**7.** Cellule, de préférence cellule mammifère, plus préférablement une cellule Vero, contenant le vecteur recombinant d'ORFV selon l'une des revendications précédentes.

**8.** Composition, de préférence composition pharmaceutique, contenant le vecteur recombinant d'ORFV selon l'une des revendications 1 à 6 et/ou la cellule selon la revendication 7.

**9.** Composition selon la revendication 8, **caractérisée en ce qu'**il s'agit, pour la composition pharmaceutique, d'un vaccin, de préférence d'un vaccin polyvalent.

**10.** Utilisation du vecteur recombinant d'ORFV selon l'une des revendications précédentes 1 à 6 pour une expression d'au moins un gène étranger, à l'exception pour une expression *in vivo.*

**11.** Utilisation selon la revendication 10 pour la préparation d'un vaccin contenant au moins un produit génique étranger, qui est un vaccin monovalent, de préférence d'un vaccin contenant au moins deux produits géniques étrangers, qui est un vaccin polyvalent.

**Fig. 1**

**Fig. 2**

**(A)** D1701-V-P2Cherry

**(B)** D1701-V-GFP-P2Cherry | D1701-V-GFP-P2Cherry | D1701-V-GFP-P2Cherry | D1701-V-GFP-P2Cherry

GFP | mCherry | merged | Durchlicht

**(C)** D1701-V-P1GFP-P2Cherry

**Fig. 3**

EP 3 325 631 B1

Fig. 4

18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **RZIHA et al.** Generation of recombinant parapoxviruses: non-essential genes suitable for insertion and expression of foreign genes. *Journal or Biotechnology*, 2000, vol. 83, 137-145
- **BÜTTNER ; RZIHA**. Parapoxviruses: From the Lesion to the Viral Genome. *J. Vet. Med. B.*, 2002, vol. 49, 7-16
- **NING et al.** BMC Veterinary Research,. *Generation of recombinant Orf virus using an enhanced green fluorescent protein reporter gene as a selectable marker*, 2011, vol. 7 (80), 1-13
- **TAN et al.** Surface display of Echinococcus granulosus antigen EG95 by fusion to membrane structural proteins. *Vaccine, Development of orf virus as a bifunctional recombinant Vaccine*, 2011, vol. 30, 398-406
- **COTTONE et al.** Analysis of genomic rearrangement and subsequent gene deletion of the attenuated Orf virus strain D1701. *Virus Research*, 1998, vol. 56, 53-67
- **DAVIDSON ; MOSS**. Structure of vaccinia virus late promotors. *J. Mol. Biol.*, 1989, vol. 210, 771-784
- **YANG et al.** Genome-wide analysis of the 5' and 3' ends of Vaccinia Virus early mRNAs delineates regulatory sequences of annotated and anomalous transcripts. *J. Virology*, 2011, vol. 85 (12), 5897-5909
- **BROYLES**. Vaccinia virus transcription. *J. Gen. Virol.*, 2003, vol. 84 (9), 2293-2303